(19) **Europäisches Patentamt**
**European Patent Office**
**Office européen des brevets**

(11) **EP 3 936 864 A1**

(12) **EUROPEAN PATENT APPLICATION**

(43) Date of publication:
**12.01.2022 Bulletin 2022/02**

(51) Int Cl.:
**G01N 33/50** (2006.01)      **G01N 33/543** (2006.01)

(21) Application number: **20382602.9**

(22) Date of filing: **06.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Consejo Superior de Investigaciones Científicas (CSIC)**
  **28006 Madrid (ES)**
• **Universidad Autónoma de Madrid**
  **28049 Madrid (ES)**
• **Immunostep, S.L.**
  **37007 Salamanca (ES)**

(72) Inventors:
• **VALÉS GÓMEZ, María del Mar**
  **28049 Madrid (ES)**
• **CAMPOS SILVA, Carmen**
  **28049 Madrid (ES)**
• **CÁCERES MARTELL, Yaiza**
  **28049 Madrid (ES)**
• **REYBURN, Hugh Thomson**
  **28049 Madrid (ES)**
• **YÁÑEZ MÓ, María**
  **28049 Madrid (ES)**
• **JARA ACEBEDO, Ricardo**
  **37007 Salamanca (ES)**

(74) Representative: **Pons**
  **Glorieta Rubén Darío 4**
  **28010 Madrid (ES)**

(54) **METHOD FOR THE DETECTION AND/OR QUANTIFICATION OF EXTRACELLULAR VESICLES IN FLUID BIOLOGICAL SAMPLES**

(57)     The invention relates to a method for detecting and/or quantifying extracellular vesicles (EVs) which are present in a biological fluid sample using a solid surface functionalized with a capture molecule, a detection molecule and polycationic polymers. The method of the invention is useful for the detection and/or quantification of biomarkers present in the surface of the EVs as well as in the diagnostic of diseases.

**EP 3 936 864 A1**

**Description**

The invention relates to a method for detecting and/or quantifying extracellular vesicles (EVs) which are present in biological fluid samples using a solid surface functionalized with an EVs capturing molecule. The method of the invention is useful for the detection and/or quantification of disease biomarkers present on the surface of the EVs as well as in the diagnostic of diseases.

## BACKGROUND OF THE INVENTION

During their normal life cycle, cells can release different types of extracellular vesicles (EVs) that originate from a variety of processes involving membrane invaginations and pinching-off events. Thus, different types of extracellular vesicles can be found in biological fluids, providing information about the different physio-pathological processes occurring in any individual. These vesicles allow trafficking of diverse subcellular components as mediators to the exterior milieu. Exosomes are a subtype of EV generated from the cellular endocytic pathway, which select and carry different types of biomolecules, from nucleic acids to proteins and metabolites, depending on the physiological state of the originating cell. For this reason, they are regarded as a potentially useful tool displaying biomarkers and so, they have attracted recently great attention from scientists, clinicians, and companies.

Nowadays much research effort is being invested to understand the biological roles of circulating EVs, to identify their origin (distinguishing those from healthy cells from those associated with pathology) and to unveil their use as biomarkers. Progress in these research areas depends on the ability to systematically characterize EVs using standardized, quantitative methods that allow comparison of results obtained in different laboratories and hospitals.

Recent works have provided techniques for the detection and isolation of EVs from biological fluids. The document WO2013158203 discloses methods and compositions using volume-excluding polymers such as polyethylene glycols (PEG) to precipitate exosomes from biological fluids. The document WO2018070479 discloses a method for recovering EVs based on treating a sample with a chelating agent, extracting the chelating agent from the treated sample and obtaining the EVs bound to the chelating agent. Both these methods, and similar ones, work mainly as enrichment methods for EVs from biological fluids that allows other methods and techniques to be carried out in the enriched solution to better detect, quantify and/or isolate specific EVs. The document WO2017198695 provides methods and kits for both the enrichment step and the specific detection of EVs from biological fluids. The methods disclosed in the document WO2017198695 are based on contacting the biological fluid sample with a polycationic substance coated on a solid surface, to which the EVs will bind. This method allows for the global enrichment in EVs in the final solution. WO2017198695 further discloses a method whereby the bound EVs to the polycationic polymer are specifically detected and/or isolated by adding an antibody to the solution, antibody which is then detected or extracted from the solution.

Despite these recent advances, for the identification of EV markers associated with different pathologies it is still necessary to design high throughput studies that allow comparison of the values of a patient cohort with those of healthy donors in order to detect biomarkers for the different pathologies. With the current methods available this type of studies can only be performed in a research laboratory as detection requires either relatively large volume samples or long protocols of EVs pre-enrichment and the use of sophisticated, specialized techniques. The recent development of nanosensors has provided a tool to design studies in a large number of patients, however, they require purpose-designed devices to be used in specialized laboratories and thus, these studies are still scarce.

Therefore, alternative methods for the detection and/or quantification of EVs that allow the simultaneous detection of specific markers on surface of EVs are required to address the aforementioned limitations of existing methods.

## DESCRIPTION OF THE INVENTION

The problems involved in carrying out large screening projects in any research or clinical setting in the field of EVs centers on the need to develop simple techniques that can be used widely and which allow the identification of extracellular vesicles in small sample volumes and with minimal manipulation. Such requirements allow for the detection and/or quantification of EVs to be done rapidly without requiring specialized personnel or equipment. To address the limitations in the existing methods and requirements set forth, the present invention discloses a method for the detection and/or quantification of EVs directly in fluid biological samples which can be carried out by personnel performing routine analysis in the clinics and requires minimal specialized equipment.

The method described in the present invention allows the direct detection of EVs, preferably exosomes, in biological fluid samples, improving the efficiency of EVs detection in immunocapture assays, which allows the study of large panels of samples with minimal effort and minimal sample volume in any laboratory setting. Cationic polymers used in this method improve detection of EVs and scarce proteins present in them. On the other hand, reducing the final volume of the sample to be assayed increases the sensitivity of EVs detection and allows the possibility of performing the assay in multi-well platforms opening the possibility of automatization.

Finally, the method of the present invention avoids the need of large sample preprocessing and EVs enrichment.

Therefore, a first aspect of the present invention relates to a method for the detection and/or quantification of EVs from a biological fluid sample, from here onwards the method of the invention, comprising:

a) Obtaining a biological fluid sample of volume between 5 and 45 μL;
b) Contacting the biological fluid sample obtained in a) to a solid surface functionalized with at least one capturing molecule capable of binding EVs, obtaining a first solution;
c) Incubating the first solution obtained in step b) with at least one detection molecule capable of binding to an epitope in the EVs; and
d) Detecting and/or quantifying the detection molecule from step c) bound to the epitope of EVs.

The terms "detection", "detecting" or "identification" as used herein refer to the finding out of the existence, presence, or absence in the fluid sample of EVs in general or of specific EVs which contain at their surface the target for the capturing molecule used in step b) and the detection molecule used in step c) of the method of the invention.

The terms "quantification" or "quantifying" as used herein refer to the determination of the relative quantity, number, or concentration of the object being quantified in comparison with the total quantity, number, or concentration of EVs in the same sample, between different biological fluid samples, or EVs bound to an isotype control antibody, wherein the isotype control antibody is used as the capture molecule of step b) or as the detection molecule of step c) of the method of the invention. In the case of the method of the invention, said object are EVs in general or specific EVs. Methods of quantification of EVs will be dependent on the detection molecule used and will be described further along in this description.

The term "extracellular vesicle (EV)" refers to a membrane-structured vesicle, in which the vesicle inner and outer regions are separated by a lipid bilayer, secreted from a cell and released into the extracellular space. EVs have plasma membrane lipids, plasma membrane proteins, nucleic acids, and cytoplasmic components, which indirectly determine the nature and state of the cells of their origin. EVs act as an extracellular carrier that mediates cell-cell communication by transferring membrane components, nucleic acids like mRNAs and miRNAs, proteins (growth hormone, cytokines, etc.). As used herein the term EV does not have any restriction or exclusion criteria based on size (in nanometer), markers (e.g. surface marker) or release mechanism (e.g. MVB- derived).

Several types of EVs exist, being the major types exosomes, microvesicles and apoptotic bodies. Accordingly, in a preferred embodiment of the method of the invention the EVs are exosomes. The term "exosome" as used herein refers to small secreted vesicles (typically about 30-150 nm) which may contain, or have present in their membrane, nucleic acid, protein, or other biomolecules and may serve as carriers of this cargo between diverse locations in a body or biological system.

The method of the invention is capable of detecting EVs from a sample of biological fluids as these fluids act as the extracellular milieu to which the cells secrete EVs. As such, term "biological fluid" as used herein means any fluid isolated or derived from an organism including prokaryotes, eukaryotes, bacteria, fungi, yeast, invertebrates, vertebrates, reptiles, fish, insects, plants and animals. Media taken from cultured cells ('conditioned media', cell media, cell culture media) is also encompassed by the term "biological fluid". EVs can therefore be isolated from a wide variety of sources of biological fluids including mammals such as mice, rats, guinea pigs, rabbits, dogs, cats, bovine, horses, goats, sheep, primates, or humans. Therefore, in a preferred embodiment of the method of the invention the biological fluid sample is obtained from a mammal, preferably a human. In another preferred embodiment of the invention the biological fluid sample is selected from the list consisting of: urine, serum, plasma, amniotic liquid, cerebrospinal fluid, ascites, bronchoalveolar wash, synovial liquid, breast milk, saliva, sweat, bone marrow and blood.

The term "sample" as used herein refers to a small part or quantity of a biological fluid that is considered representative of its total and that is taken or separated from it in order to submit it to study, analysis, or experimentation. In the present invention, such study, analysis, or experimentation refers to the detection and/or quantification of EVs by the method of the invention.

The expression "obtaining a biological fluid sample of volume between 5 and 45 μL" in step a) of the method of the invention, refers to the process of collecting a biological fluid sample from a subject. Methods, techniques, and best practices to obtain biological fluid samples from subjects are well known by the expert (e.g., clinicians), who will be aware of such methods, techniques, and best practices and how best to employ them.

The use of a minimal biological fluid sample volume relates to the discovery that a smaller biological fluid sample leads to an increase in the sensitivity of the method of the invention.

The inventors further discovered that the presence of cationic polymers in the fluid sample leads to the increase in the zeta potential leading to flocculation of the EVs present in the sample, which in turns leads to a further increase of the sensitivity of the method of the invention. Therefore, in a preferred embodiment of the method of the invention a cationic polymer is added to the biological fluid sample obtained in step a).

The expression "a cationic polymer is added to the biological fluid sample obtained in step a)" refers to the process of mixing, combining, or contacting the biological fluid sample obtained in step a) of the method of the invention with a cationic polymer. The term "cationic polymer" as used herein refers to a positively charged polymer. The cationic polymer molecule can be composed of several monomers and have positive charges in multiple places. In a preferred embodiment of the method of the invention, the cationic polymer is added to the biological fluid sample at a final concentration of 1 to 20 μg/ml, preferably between 4 μg/ml and 8 μg/ml. In another preferred embodiment of the method of the invention, the cationic polymer has a molecular weight of 4-300 kDa. In a further preferred embodiment, the cationic polymer induces an increase in the zeta potential value of the EVs solution or biological fluid.. In another preferred embodiment of the invention, the cationic polymer added to the biological fluid sample is selected from the list consisting of: poly-L-lysine, hexadimethrine bromide (also known as polybrene), gelatin, acrylamide chloride, diallyldimethylammonium chloride, cationic chitosan, cationic cellulose, cationic cyclodextrin, polyethyleneimine, polyamidoamines, polyamino-co-ester, polydimethylaminoethylmethacrylate and cationic dextran, preferably poly-L-lysine and hexadimethrine bromide.

The term "zeta potential" as used herein refers to a measure of the surface charge of the nanoparticles. The zeta potential is determined for suspended nanoparticles in an electrolyte. In this suspension, an ion distribution of the electrolyte is produced around the surface of the nanoparticle. A first monolayer of oppositely charged ions attached electrostatically to the nanoparticle and a zone around surrounding solution having an excess of ions opposite the nanoparticle, outside this zone the concentration of ions is constant at all points and equal to that of the electrolyte. The potential difference between the monolayer and the envelope is the zeta potential. The zeta potential determines the degree of repulsion between adjacent nanoparticles of charge of the same sign. If it falls below a certain value, the forces of attraction exceed those of repulsion and the nanoparticles aggregate. The potential was measured in solutions of the particles diluted in Phosphate-Buffered Saline (PBS) buffer, measuring the electrophoretic mobility in a Zetaview equipment.

Having obtained the biological fluid sample, the next step of the method of the invention refers to forming complexes between the EVs and a solid surface which has on its surface at least one molecule capable of capturing the EVs or a molecule on the EVs surface. Therefore, the expression "contacting the biological fluid sample obtained in a) with a solid surface functionalized with at least one capturing molecule capable of binding EVs" of step b) of the method of the invention refers to such process of bringing the biological fluid sample, with or without a cationic polymer, into contact with a solid surface that has on its surface at least one agent or molecule that can recognize the EVs or a target molecule in the surface of the EVs and bind to such target, forming stable bound complexes between the capture molecule and the target EV or molecule. Said process of "contacting the biological fluid sample with a functionalized solid surface" is performed at a temperature and in an interval of time sufficient for the forming of stable bound complexes between the EVs and the capture molecule. In a preferred embodiment step b) of the method of the invention is performed at room temperature. In another preferred embodiment step b) of the method of the invention is performed during an interval of time between 1 and 24 hours, preferably at least 2, 3, 4, 5, 6, 7, 8, 9, 10, 11, 12, 13, 14, 15, 16, 17, 18, 19, 20, 21, 22, 23 hours.

The term "solid surface" a used herein refers to a material having a rigid or semi-rigid surface to which a compound or a molecule can be attached or upon which they can be synthesized. A solid surface can be found on or in a solid-phase support or substrate. In another preferred embodiment of the invention, the solid surface is selected from the group consisting of: resin, microparticles, glass, plastic, membrane, plastic beads and glass beads. In a more preferred embodiment, the microparticles are selected from polymer microparticles, silica microparticles, magnetic microparticle or latex microparticles. In another embodiment, the polymer microparticles are selected from polystyrene microparticles or methacrylate microparticles.

The term "solid surface functionalized" as used herein refers to the coating, binding or conjugation of the solid surface with functional groups or molecules that allow the binding of the solid surface to a specific target, namely an EV or a specific molecule, e.g. a protein, lipid, phospholipid, at the surface of the EV. Several methods are known in the art for the functionalization of solid surfaces, and the expert in the field would be aware of such methods. Examples of such methods are, without limitation, temporary swelling of microparticles in order to have the hydrophobic part of the functionalizing molecules to become trapped inside the microbeads upon deswelling, covalent attachment of the functionalizing molecules to the amine groups present at the surface of the solid, click chemistry techniques, simple adsorption of protein onto microparticles, carboxylate covalent coupling chemistries and chloromethyl-activation.

In a preferred embodiment of the method of the invention, the functionalization of the solid surface is performed by simple adsorption of capturing molecules to an ELISA plate or by high density carboxyl functional groups on the surface of microparticles. Said carboxyl functional groups are used to covalently conjugate antibodies through their primary amines carbodiimide crosslinker chemistry to produce a stable amide bond.

The term "capturing molecule" as used herein refers to any substance as long as it has the property of binding or attaching to EVs. The capturing molecule may be one that recognizes and/or binds the EV or a specific target in the surface of the EVs allowing the capture of the EVs or the EVs that possess the specific target in their surface. In another preferred embodiment of the method of the invention, the capture molecule functionalized to the solid surface is selected from a list consisting of: annexin, lectins and immunoglobulins, preferably wherein the immunoglobulin is an antibody. In another preferred embodiment the capture molecule is an aptamer. In a more preferred embodiment, the antibody is selected from the list consisting of: anti-CD63, anti-CD81, anti-CD9, anti-tetraspanins, anti-EpCAM, anti-MICA, anti-PD-L1 antibody.

The term "annexin" as used herein refers to a group of proteins capable of binding to negatively charged phospholipids in a calcium dependent manner and that contain a 70 amino acid repeat sequence called an annexin repeat. The term "annexin" includes a natural annexin purified from a natural source such as a human placenta, or an annexin molecule comprising a native sequence produced by, for example, genetic engineering or other methods. Unless otherwise specified, the term annexin includes annexin derived from any source. In a preferred embodiment of the method of the invention, the annexins are selected from a list consisting of: annexin V, A1, A2, A4 and A5.

The term "lectin" as used herein refers to a sugar-binding protein of non-immune origin that agglutinates cells or precipitates glycoconjugates. The lectin molecule contains at least two sugar-binding sites; sugar-binding proteins with a single site will not agglutinate or precipitate structures that contain sugar residues, so are not typically classified as lectins. The specificity of a lectin is usually defined by the monosaccharides or oligosaccharides that are best at inhibiting the agglutination or precipitation the lectin causes. Lectins occur in many types of organism, they may be soluble or membrane-bound, they may be glycoproteins. Sugar-specific enzymes, transport proteins and toxins may qualify as lectins if they have multiple sugar binding sites. Examples of lectins are polylactosamine-binding lectins, polylactosamine-binding lectins and galactose-specific lectins. In a preferred embodiment of the method of the invention, the lectins are selected from a list consisting of: *Solanum tuberosum* lectin, *Sambucus nigra* agglutinin lectin, *Sambucus sieboldiana* agglutinin lectin, *Trichosanthes japonica* agglutinin II lectin, galectin 1 and galectin 3.

The term "immunoglobulin" as used herein refers to an extensive group of soluble and cell surface proteins that are involved in processes of cell recognition, binding or adhesion of cells. The assignment of a molecule to this superfamily is based on the fact that they share structural features with immunoglobulins (also known as antibodies). All of them possess a domain known as immunoglobulin domain or folding. Immunoglobulins group include cell surface antigen receptors, correctors, and co-stimulation molecules of the immune system, molecules involved in antigen presentation to lymphocytes, cell adhesion molecules, and certain cytokine receptors. Examples of immunoglobulins are antibodies and Proteins of the transmembrane immunoglobulin and mucin domain (TIM) family.

The term "antibody" as used herein refers to a protein that belongs to the immunoglobulin proteins, that is produced in response to an antigen that is able to detect and bind to the antigen, preferably at a specific site on the antigen, known as an epitope. The term as used herein includes antibodies of polyclonal and monoclonal origin, unless stated otherwise. Polyclonal antibodies are a group of antibodies produced by different B lymphocytes in response to the same antigen; different antibodies in the group typically recognize different parts (epitopes) on the antigen. A monoclonal antibody recognizes only one type of antigen and is produced by the daughter cells of a single antibody-producing lymphocyte, typically a hybridoma. Also included within the term 'antibody' are antigen binding fragments of naturally or non-naturally occurring antibodies, for example, the "Fab fragment", "Fab' fragment" (a Fab with a heavy chain hinge region) and "F(ab')2 fragment" (a dimer of Fab' fragments joined by a heavy chain hinge region). Recombinant methods have been used to generate small antigen-binding fragments, such as "single chain Fv" (variable fragment) or "scFv," consisting of a variable region light chain and variable region heavy chain joined by a synthetic peptide linker. Unlike antibodies derived from other mammals, camelid species express fully functional, highly specific antibodies that are devoid of light chain sequences. Camelid heavy chain antibodies are of particular use, as they are found as homodimers of a single heavy chain, dimerized via their constant regions. The variable domains of camelid heavy chain antibodies are referred to as VHH domains and retain the ability, when isolated as small fragments of the VH chain, to bind antigen with high specificity. Further included within the term 'antibody' are so called camelized mutants of human VH domains that retain antigen binding activity but exhibit some of the advantages of camelid VHH domains.

The term "aptamer" as used herein refers to a single-stranded nucleic acid molecule (e.g. DNA or RNA) or peptide having a function that allows the nucleic acid molecule or peptide to specifically bind to a specific substance such as a protein, an organic small molecule, a nucleic acid or a multimeric complex in the EVs or to the EVs themselves. An "aptamer" can bind to its target with high affinity, in the picomolar range.

In addition to the capture molecule, which is directly bound to a solid surface, a "detection" molecule recognizing an EV epitope in the EV surface is used in the assay. Therefore, the expression "incubating the solution obtained in step b) with a detection molecule capable of binding to an epitope in the EVs" in step c) of the method of the invention refers to the process of adding or mixing a compound that has the capability of identifying and bind to an epitope in the surface of EVs. Said "incubation" process is performed at a temperature and in an interval of time sufficient for the stable binding of the detection molecule to its target on the EVs. In a preferred embodiment step c) of the method of the invention is performed at 4 °C. In another preferred embodiment step c) of the method of the invention is performed during an interval of time between 0.5 and 4 hours, preferably between 1 and 2 hours, more preferably for 1 hour.

The term "detection molecule" as used herein refers to the molecule used in step c) of the method of the invention which binds to an epitope in the surface of EVs immobilized onto the solid surface through the capture molecules.

In a preferred embodiment of the method of the invention, the detection molecule is selected from a list consisting of: annexin, lectins and immunoglobulins, preferably wherein the immunoglobulin is an antibody. I another preferred embodiment the detection molecule is an aptamer. In another preferred embodiment the antibody is selected from the list consisting of: anti-CD63, anti-CD81, anti-CD9, anti-tetraspanins, anti-EpCAM, anti-MICA, anti-PD-L1 antibody. In yet another preferred embodiment the annexin is selected from a list consisting of: annexin V, A1, A2, A4 and A5. In another preferred embodiment the lectin is selected from a list consisting of: *Solanum tuberosum* lectin, *Sambucus nigra* agglutinin lectin, *Sambucus sieboldiana* agglutinin lectin, *Trichosanthes japonica* agglutinin II lectin, galectin 1 and galectin 3.

The terms "lectin", "annexin", "aptamer", "immunoglobulin" and "antibody" have been previously defined in this description in relation to the capture molecule of step b) of the method of the invention, and such definitions are valid in relation to the detection molecule of step c) of the method of the invention.

In a preferred embodiment of the method of the invention the capture molecule that functionalized to the solid surface and the detection molecule are different.

The term "detection molecule" also includes molecules bound or conjugated to labels or markers. As an example, the detection molecule can be a chimeric fusion between an antibody and a label protein such as the green fluorescent protein (GFP). Such labels or markers do not alter the function of detecting and/or quantifying EVs. As such, in another preferred embodiment the detection molecule of step c) of the method of the invention is conjugated to a detectable label. Such "conjugation" may be performed by any known method to functionally connect the domains of a detection molecule with a marker that produces a signal capable of being visually detected, including, without limitation, recombinant fusion with or without intermediate domains, antibody-mediated fusion, non-covalent association and covalent bonding, e.g., antibody to disulfide bonding, hydrogen bonding, electrostatic bonding and conformational bonding, e.g., biotin-avidin binding. Conjugation with a marker can occur by chemical or recombinant processes. Chemical processes refer to a reaction between the molecule and the label or marker in such a way that a covalent bond is formed between the two molecules to form a single molecule.

The term "detectable label" or "marker" as used herein, refers to molecules that can generate a signal that is detectable by a method suitable for such detection. Marker detection methods include, without limitation, fluorescence, optical, confocal and electron microscopy, magnetic resonance and spectroscopy; fluoroscopy, computed tomography and positron emission tomography, enzyme-linked immunosorbent assays, fluorescence detection size exclusion chromatography, lateral flow assay, radioimmunoassay, radio-labelling, western blotting, immunohistochemistry, immunofluorescence, immunocytochemistry, flow cytometry, fluorescence-activated cell sorting, mass cytometry, multiomic cytometry, immunoprecipitation and enzyme-type immunospots. Suitable markers include, but are not limited to, fluorescein, rhodamine, eosin and other fluorophores, radioisotopes, gold, gadolinium and other lanthanides, paramagnetic iron, fluorine-18 and other positron-emitting radionuclides, oligonucleotides and heavy metal isotopes. In addition, the probes or labels may be bi or multi-functional and detectable by more than one of the methods listed. The marker or label may be, for example, a chromogenic, fluorogenic, magnetic, electrodense, radioactive and/or chemiluminescent compound. In another preferred embodiment of the present invention, the detectable label or marker is selected from the list consisting of: phycoerythrin (PE), fluorescein isothiocyanate (FITC), allophocyanine (APC) or any other fluorescent dye relevant in flow cytometry, biotin, horseradish peroxidase (HRP), gold beads and paramagnetic iron.

The term "epitope" as used herein refers to a physical structure on a molecule that interacts with a selective component. In the present invention such physical structure is on a molecule that is on the surface of EVs and the selective component is the detection molecule used in step c) of the method of the invention.

After EV binding to the functionalized solid surface, EVs or a target molecule on the surface of EVs can be detected and/or quantified. Therefore, the process of detecting and/or quantifying the EVs that form a complex with the capture molecule refers to the expression "detecting and/or quantifying the detection molecule bound in step c)" in step d) of the method of the invention. In a preferred embodiment, the detection in step d) of the method of the invention is made by flow cytometry, multiplex flow cytometry-based instruments compatible with multiwell plates or any other laser detection instrument, preferably flow cytometry.

One of the main advantages of the method of the invention is the small volume of biological fluid sample required to carry out the method. Therefore, the method of the invention is ideally suited to be automatized, since it allows the use of multiwell plates (e.g. micro 96-well plates), using commercially available automated systems, known to the expert in the field. Such automatization will allow effortless sample processing by personnel and permit easy scaling up of subject screenings. As such, in another preferred embodiment of the method of the invention the method is carried out in a micro-well container.

The term "container" as used herein refers to any container, carrier or support acceptable to perform the method of the invention. The container can be made of glass, plastic, ceramic material, metal or any other material commonly used to perform such methods or store the reagents of the method of the invention. However, an acceptable material should not react with the contents it is intended to hold.

The method of the present invention as described above finds use in two main fields, both linked to the detection and/or quantification of the detection molecule bound to the epitopes on the EVs surface, namely the *in vitro* diagnosis of diseases and the detection and/or identification of biomarkers of a disease displayed by EVs.

As such, another aspect of the present invention relates to an *in vitro* method for the diagnosis of a disease, from here onwards the diagnostic method of the invention, which comprises the steps of the method of the invention:

a) Obtaining a biological fluid sample of volume between 5 and 45 μL;
b) Contacting the biological fluid sample obtained in a) to a solid surface functionalized with at least one capturing molecule capable of binding EVs or a disease biomarker, obtaining a first solution;
c) Incubating the first solution obtained in step b) with at least one detection molecule capable of binding to an epitope in the EVs; and
d) Detecting and/or quantifying the detection molecule from step c) bound to the epitope of EVs.

wherein the binding by the capturing molecule to a disease biomarker in the step b) or the binding by the detection molecule to an epitope in the EV from step c), wherein the epitope is an epitope of a disease biomarker, indicates the presence of the disease.

The terms "capture molecule", epitope", "biomarker" and "EV" have been previously described and defined in this description and said definitions and descriptions are valid for the present aspect of the invention.

As used herein, the term "diagnosis", refers to ascertaining by the method of the invention the presence or absence of a specific molecule or molecules whereby the presence or absence of such molecule or molecules is indicative of the existence of a pathological condition in the subject form where the biological fluid sample originates. In addition, the term "diagnosis" further includes the quantification of the relative numbers, concentration or levels of such molecule/molecules in comparison to the levels of samples from healthy subjects, wherein their altered levels is indicative of the existence of a pathological condition in the subject from where the biological fluid sample originates. In a preferred embodiment of the diagnostic method of the invention, the disease is selected from the list consisting of: cancer, autoimmune diseases, inflammatory diseases, immunodeficiencies, infectious diseases, pathogen originated diseases, cardiovascular diseases, degenerative diseases, hormone disorder related diseases, and metabolic diseases. In a more preferred embodiment the disease is diabetes or Alzheimer's disease.

The terms "autoimmune diseases" or "inflammatory diseases" as used herein refer to a condition arising from an abnormal immune response to a normal body part. Examples of such diseases are Type 1 diabetes, Rheumatoid arthritis (RA), Psoriasis/psoriatic arthritis, Multiple sclerosis, Systemic lupus erythematosus (SLE), Inflammatory bowel disease (Crohn's disease and Ulcerative colitis), Addison's disease, Graves' disease, Sjögren's syndrome, Hashimoto's thyroiditis, Myasthenia gravis, Autoimmune vasculitis, , allergy, asthma, Celiac/coeliac disease, glomerulonephritis, hepatitis, reperfusion injury and transplant rejection.

The term "immunodeficiencies" as used herein refers to a diverse group of conditions characterized chiefly by an increased susceptibility to various infections with consequent severe acute, recurrent and chronic disease which result from one or more defects in the specific or nonspecific immune systems. Examples of immunodeficiencies are Autoimmune Lymphoproliferative Syndrome (ALPS), APS-1 (APECED), BENTA Disease, Caspase Eight Deficiency State (CEDS), CARD9 Deficiency and other syndromes of susceptibility to Candidiasis, Chronic Granulomatous Disease (CGD), Common Variable Immunodeficiency (CVID), Congenital Neutropenia Syndromes, CTLA4 Deficiency, DOCK8 Deficiency, GATA2 Deficiency, Glycosylation Disorders with Immunodeficiency, Hyper-Immunoglobulin E Syndromes (HIES), Hyper-Immunoglobulin M Syndrome, Interferon Gamma, Interleukin 12 and Interleukin 23 Deficiencies, Leukocyte Adhesion Deficiency (LAD), LRBA Deficiency, PI3 Kinase Disease, PLCG2-associated Antibody Deficiency and Immune Dysregulation (PLAID), Severe Combined Immunodeficiency (SCID), STAT3 Dominant-Negative Disease, STAT3 Gain-of-Function Disease, Warts, Hypogammaglobulinemia, and Myelokathexis (WHIM) Syndrome, Wiskott-Aldrich Syndrome (WAS), X-Linked Agammaglobulinemia (XLA), X-Linked Lymphoproliferative Disease (XLP), and XMEN Disease.

The terms "infectious diseases" and "pathogen originated diseases" relate to diseases causes by organisms such as viruses, bacteria, fungi and parasites. Examples of such diseases are flu, COVID19, Severe Acute Respiratory Syndrome (SARS), Middle East respiratory syndrome (MERS), Human Immunodeficiency Virus (HIV) Acquired Immune Deficiency Syndrome (AIDS), smallpox, zica disease, cholera, dengue fever, leprosy, Lyme disease, Ebola disease, leishmaniasis, malaria, tuberculosis, candidiasis, Mycosis, gonorrhea, syphilis, chlamydia, trichomoniasis, human papillomavirus and genital herpes .

The term "cardiovascular disease" as used herein refers to a class of diseases that involve the heart or blood vessels. Examples of such diseases are angina and myocardial infarction, stroke, heart failure, hypertensive heart disease, rheumatic heart disease, cardiomyopathy, abnormal heart rhythms, congenital heart disease, valvular heart disease, carditis, aortic aneurysms, peripheral artery disease, thromboembolic disease, venous thrombosis. coronary heart or artery disease, cerebrovascular disease, vascular disease, deep vein thrombosis and pulmonary embolism, abnormal heart rhythms, or arrhythmias, aorta disease, Marfan syndrome and Heart muscle disease (cardiomyopathy).

The term "degenerative diseases" as used herein refers to diseases which are the result of a continuous process based on degenerative cell changes, affecting tissues or organs, which will increasingly deteriorate over time. Examples of degenerative diseases are Alzheimer's disease (AD), Parkinson's disease, Amyotrophic lateral sclerosis (ALS also known as Lou Gehrig's disease), Chronic traumatic encephalopathy, Cystic fibrosis, cytochrome c oxidase deficiencies, Ehlers-Danlos syndrome, Fibrodysplasia ossificans progressiva, Friedreich's ataxia, Frontotemporal dementia (FTD), atherosclerotic disease, coronary artery disease, aortic stenosis, Huntington's disease, Infantile neuroaxonal dystrophy, Keratoconus (KC), Keratoglobus, Leukodystrophies, Macular degeneration (AMD), mitochondrial myopathies, Mitochondrial DNA depletion syndrome, Multiple sclerosis (MS), Multiple system atrophy, Muscular dystrophies (MD), Neuronal ceroid lipofuscinosis, Niemann-Pick disease, Osteoarthritis, Osteoporosis, Pulmonary arterial hypertension, Creutzfeldt-Jakob disease, fatal familial insomnia, Progressive supranuclear palsy, Retinitis pigmentosa (RP), Rheumatoid arthritis, Sandhoff Disease, Spinal muscular atrophy (SMA), motor neuron disease, Subacute sclerosing panencephalitis, Tay-Sachs disease and Vascular or degenerative dementia.

The term "metabolic diseases" as used herein refers to a group of syndromes that disrupt the normal metabolic processes in the body, namely, anabolism (forming complex molecules from simpler ones) and catabolism (forming simpler molecules from complex ones). Examples of metabolic diseases include Obesity, Hepatic lipidosis, Dysmetabolic syndrome, Hypertriglyceridemic waist, Insulin resistance syndrome, Adrenoleukodystrophy (ALD), Type I diabetes, Gaucher disease, Glucose-galactose malabsorption (GGM), Hereditary hemochromatosis, Lesch Nyhan syndrome, Maple syrup urine disease, Menkes syndrome, Phenylketonuria and Lysosomal storage disease.

The term "hormone disorders" as used herein refers to a malfunction or misfunction of the endocrine system, leading to hormonal imbalance. Examples of such disease are Type 2 Diabetes, Gestational Diabetes, Mature Onset Diabetes of the Young, Idiopathic hypoglycemia, Insulinoma, Glucagonoma, Goitre, Graves-Basedow disease, Toxic multinodular goitre, Hypothyroidism, Hashimoto's thyroiditis, Thyroid cancer, Thyroid hormone resistance, Primary hyperparathyroidism, Secondary hyperparathyroidism, Tertiary hyperparathyroidism, Hypoparathyroidism, Pseudohypoparathyroidism, Osteoporosis, Osteitis deformans (Paget's disease of bone), Rickets, Osteomalacia, Diabetes insipidus, Syndrome of Inappropriate Antidiuretic Hormone (SIADH), Hypopituitarism (or Panhypopituitarism), Pituitary adenomas, Prolactinoma (or Hyperprolactinemia), Acromegaly, gigantism, dwarfism, Cushing's disease, Hermaphroditism, Gonadal dysgenesis, Androgen insensitivity syndromes, Kallmann syndrome, Klinefelter syndrome, Turner syndrome, Ovarian failure (also known as Premature Menopause), Testicular failure, Delayed puberty, Precocious puberty, Amenorrhea, Polycystic ovary syndrome, Multiple endocrine neoplasia type 1, type 2a and type 2b, Carcinoid syndrome, Anoxic encephalopathy, Hypothyroidism, Hyperthyroidism, Hypoglycemia, Hypoparathyroidism, Hepatic failure, Renal failure and Hypernatremia and Hypomagnesemia

As referred previously, the method of the invention is ideal for the detection of biomarkers. Therefore, another aspect of the present invention is a method for the detection and/or identification of biomarkers of a disease in EVs which comprises the steps of the method of the invention and an additional step (e) in which disease biomarkers in EVs are phenotyped, from here onwards the biomarkers identification method of the invention.

The terms "detection" and "identification" were previously defined in relation to the method of the invention aspect, being such definition valid and applicable to the current aspect of the invention.

The term "biomarker" as used herein refers to a substance, the presence of which can be objectively determined and quantified, which is used as an indicator of the presence/absence of a disease or the prognosis thereof, as well as an indicator for classification of the disease or indicator of response or resistance to a treatment. The biomarker of the invention can be detected and/or quantified, that is, only its presence can be detected or changes in its relative amount or concentration can be detected and/or quantified.

The term "phenotyped" as used herein refers to the process of discovering or identifying, characterizing and validating specific biomarkers present in EVs, whose presence or whose changes in concentration, number or quantity in EVs can be associated with a specific disease, disorder or syndrome, or response to a given drug or treatment, and use for prognosis or classification of patients.

In a preferred embodiment of the biomarker identification method of the invention, the disease is selected from the list consisting of: cancer, autoimmune diseases, inflammatory diseases, immunodeficiencies, infectious diseases, pathogen originated diseases, cardiovascular diseases, degenerative diseases, hormone disorder related diseases, and metabolic diseases. In a more preferred embodiment the disease is selected from cancer, diabetes and Alzheimer's disease.

Said diseases have been previously defined in relation to the diagnosis method of the invention and such definition as well as examples of said diseases are valid for the present aspect.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

## DESCRIPTION OF THE DRAWINGS

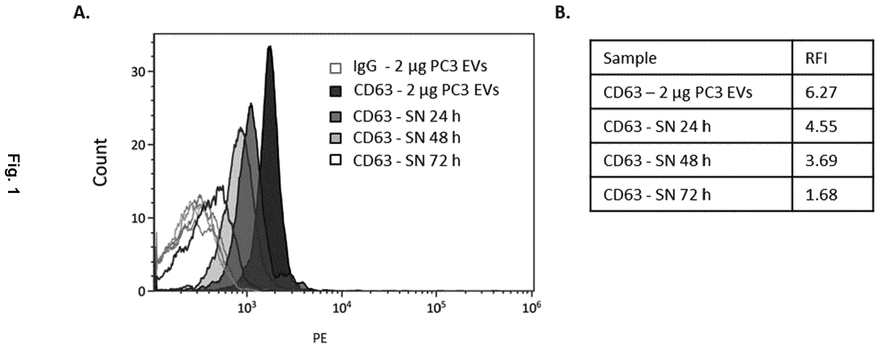

**Fig. 1. Bead-assisted immunocapture does not result in binding of all the vesicles available in the mixture.**
**A.** 6000 anti-CD63-coated beads were incubated with 2 $\mu$g of PC3-derived EVs and captured vesicles were detected by flow cytometry after incubation with anti-CD9-PE. IgG was used as a negative control. After the first incubation, supernatants containing unbound exosomes (SN 24h) were incubated with new anti-CD63 beads and EVs captured during this second incubation were analyzed by flow cytometry. This procedure was repeated the following 2 days (SN 48 h, SN 72 h). A. Histograms from a representative experiment out of 4. **B.** Relative Fluorescence Intensity (RFI) values.

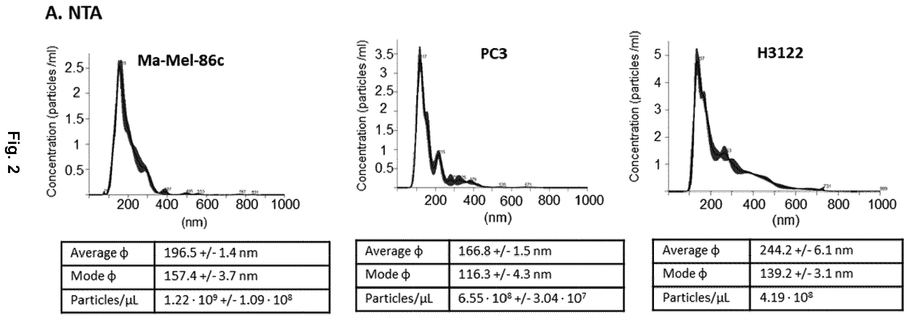

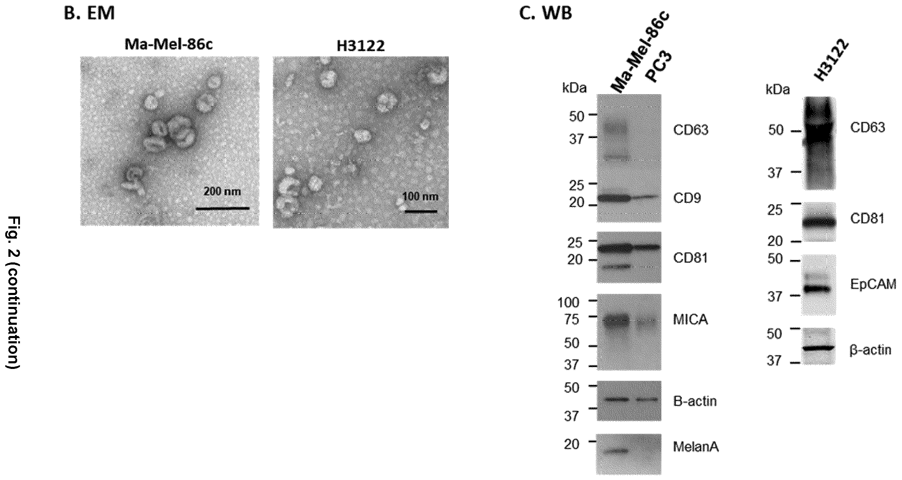

**Fig. 2. Characterization of cell lines-derived EVs.**
**A.** Size and concentration analysis by Nanoparticle tracking analysis (NTA). Average size and concentration listed in the tables were obtained in a Nanosight equipment capturing 3 videos of 60 s per measurement, with a focus -15 to +15, camera level 12 and temperature of 25 °C. Software NTA 3.1 (Malvern) was used for the analysis. $\phi$: diameter. **B.** Transmission Electron Microscopy (TEM) visualization. 1 $\mu$L EVs were diluted 1:10 in HBS and floated on a carbon-coated 400-mesh 240 Formvargrid, then incubated with 2% uranyl acetate and analyzed using a Jeol JEM 1011 electron microscope operating at 245 100 kV with a CCD camera Gatan Erlangshen ES1000W. Pictures were taken at the Electron Microscopy Facility of the CNB. Bar: 100 or 200 nm as indicated. A representative image is shown. **C.** Protein marker characterization by Western Blot. EVs were loaded in 12% SDS-PAGE gels. Membranes were immunoblotted for detection of: tetraspanins CD9, CD63, CD81 as general EV markers; $\beta$-actin as loading control; MICA, MelanA and EpCAM as cancer-related markers. For each EV type, two gels were loaded: one gel, under non-reducing conditions and the other under reducing conditions, for actin detection. One representative experiment out of 3 is shown.

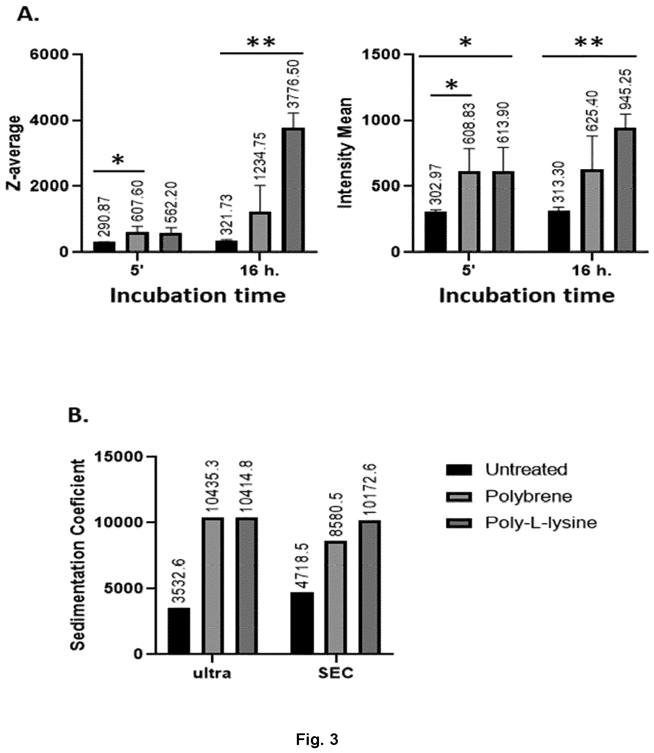

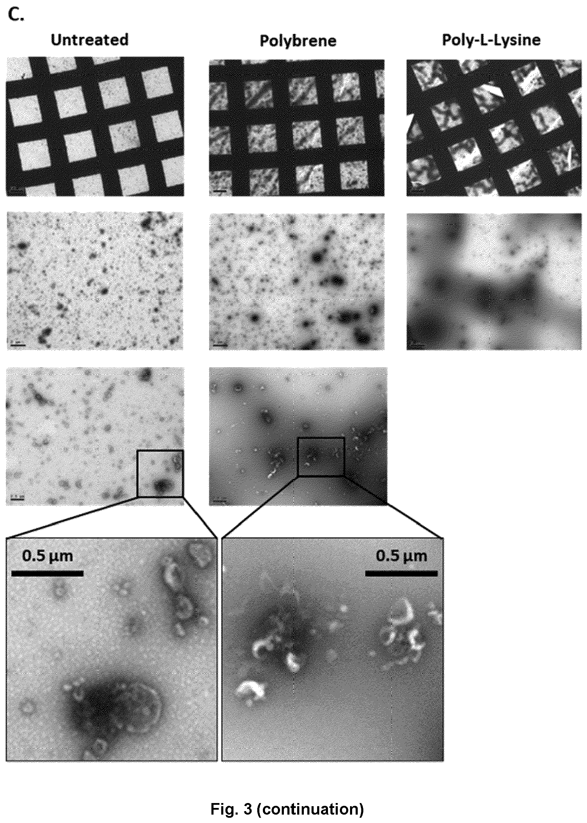

**Fig. 3. Cationic polymers affect the biophysical properties of EV suspensions and result in precipitation of the nanoparticles.**
Metastatic melanoma derived EVs were obtained by ultracentrifugation and then incubated with or without 4 $\mu$g/ml hexadimethrine bromide (also known as Polybrene) or 4 $\mu$g/ml Poly-L-lysine. **A.** Hydrodynamic diameter by Dynamic Ligh Scattering (DLS). EVs were incubated either 5 minutes or 16 h with the polymers and analyzed using a DLS instrument. Data on Z-average and Intensity Mean (average diameter) are depicted. **B.** Analytical ultracentrifugation. The sedimentation coefficient of EVs was analyzed either directly after ultracentrifugation (ultra) or after further purification by size exclusion chromatography (SEC). The graph represents data on the average sedimentation coefficient obtained after a 18 h-incubation with polymers of EVs obtained under these two conditions. **C.** Electron Microscopy. The top row shows the general aspect of the grid. Polymers caused precipitation on the grid, in fact, poly-L-Lys strongly affected the integrity of the sample and pictures at high magnifications could not be taken because the electron beam broke the resin. Bar: 2 $\mu$m (2nd raw), 0.5 $\mu$m (3rd and 4th raw).

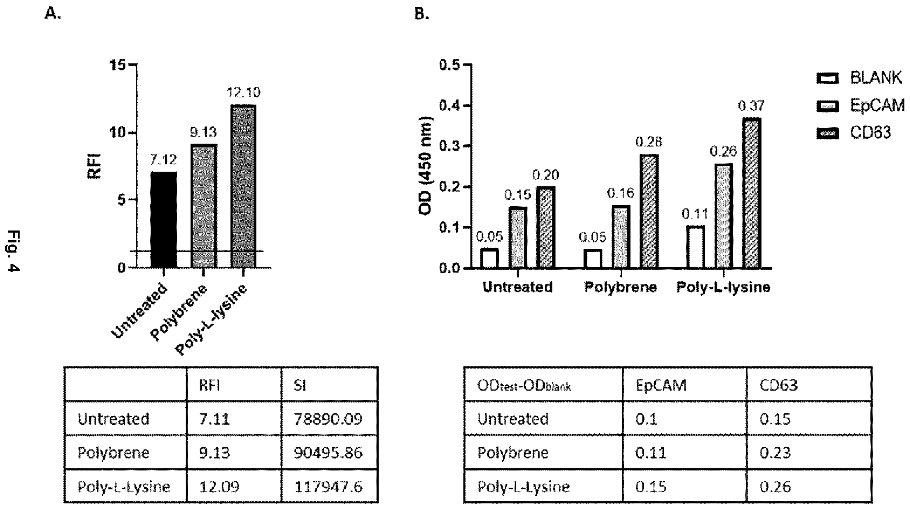

**Fig. 4. Adding Polymers increased the signal detected by flow cytometry and ELISA.**
Lung cancer-derived EVs (from H3122 cell line) were enriched by ultracentrifugation. 1.2 x 106 EVs/$\mu$l were treated with 4 $\mu$g/ml polybrene, 4 $\mu$g/ml poly-L-lysine or kept untreated for 18 h. **A.** FACS. EVs were incubated with either anti-EpCAM or IgG isotype control beads. Graphs represent the Relative Fluorescence Intensity (**RFI** = $\frac{MFI\ sample}{MFI\ IgG}$) values. RFI and Stain Index $\left(SI = \frac{(MFI\ sample - MFI\ IgG)}{(2xSD\ IgG)}\right)$ values are compared in the table below. MFI: Median Fluorescence Intensity. **B.** ELISA. Anti-CD63 or anti-EpCAM antibody-coated plates were incubated with EVs (or buffer only, for blanks). Detection was performed with biotinylated anti-CD9 antibody followed by SA-HRP. Optical Density (OD) at 450 nm values is. OD of the tested sample after blank OD subtraction is listed on the table below.

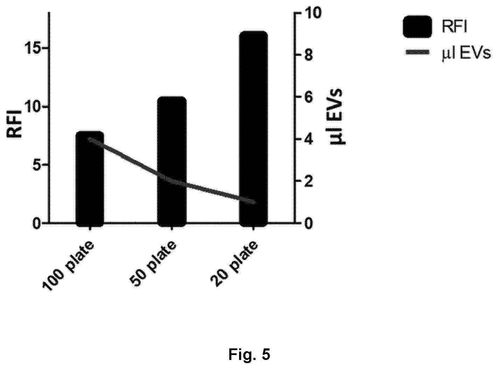

**Fig. 5. EV detection increases as volume decreases, resulting in higher sensitivity.**
**A.** Schematic representation. To compare the binding capacity of beads, assays were performed in different volumes in flat-bottom 250 $\mu$L microwells. In all cases, 3000 anti-CD63 beads were used, keeping the EV concentration constant. The volume of EVs added to each assay is represented by a grey line (right Y-axe scale). Different final volumes were used: 100 $\mu$L, 20 $\mu$l or 50 $\mu$l in plates. Lung cancer-derived EVs (cell line H3122) were obtained by ultracentrifugation, incubated with beads for 18 h and, after staining with anti-CD81-PE, washed and analyzed by flow cytometry. Lower amounts of EVs resulted in higher signal in small volume assays. Same EV concentration resulted in higher signal in small volume assays.

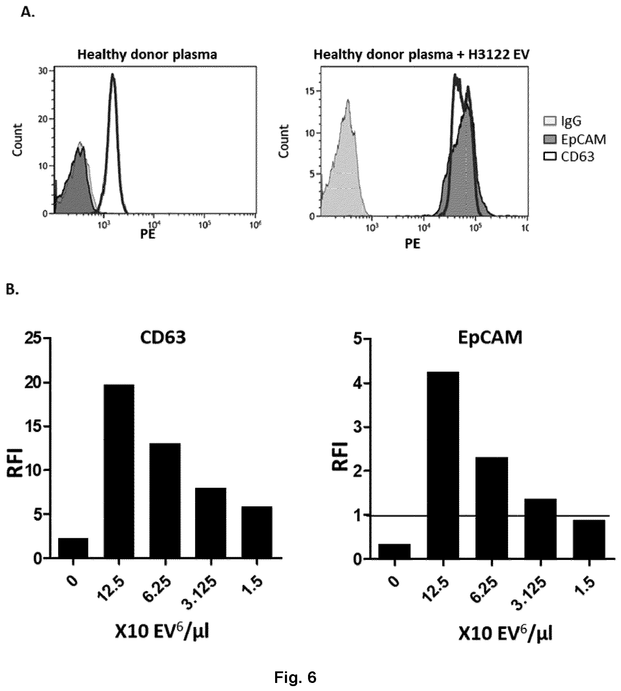

**Fig. 6. : Detection of EV proteins directly in plasma.**
12 $\mu$L of healthy donor plasma were incubated with 3000 beads in a final volume of 25 $\mu$l. EVs were captured on anti- CD63, anti-EpCAM or IgG isotype control beads as indicated in each experiment. **A.** Direct detection of tetraspanins in plasma and EpCAM from added lung cancer-derived EVs . Left. Detection of EVs directly in healthy donor plasma. No EpCAM positive EVs were observed in healthy donors, as expected. Right. $0.75 \times 10^9$ lung cancer-derived EVs (from the H3122 cell line) were added to the 12 $\mu$l of healthy donor plasma. The signal of CD63+ EVs increased compared to plain healthy donor plasma signal due to the EVs added. EpCAM-positive EVs were detected in healthy donor plasma when lung cancer-EVs were added. **B.** Limit of detection. EV proteins were analyzed by flow cytometry in plasma samples containing increasing concentrations of lung cancer-derived EVs. The limit of detection for EpCAM was below $3.125 \times 10^6$ EVs/$\mu$l.

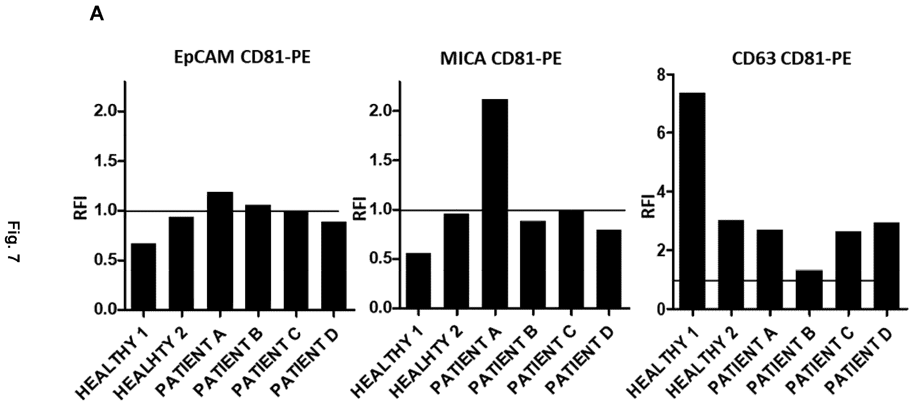

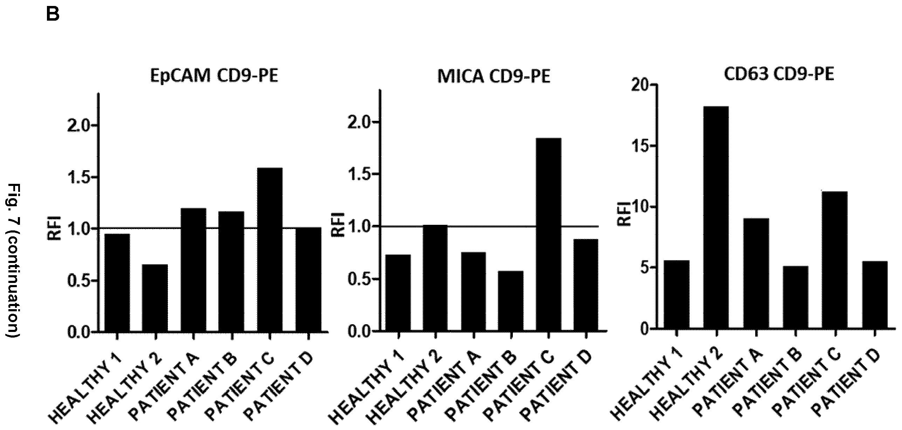

**Fig. 7. : EV tumor marker proteins. EpCAM and MICA, can be detected directly in minimal volumes of NSCLC patients' plasma.**
3000 anti-CD63, anti-MICA, anti-EpCAM or IgG isotype control beads were incubated during 18 h with 12 $\mu$l of plasma from 4 Non-Small Cell Lung Cancer patients (PATIENT A-D) and 2 healthy donors (HEALTHY 1 and 2) (final volume 25 $\mu$l). EVs captured in each assay were detected in parallel using either anti-CD9-PE (**A**) or anti-CD81-PE (**B**). Patient A had MICA and EpCAM CD81 EVs while Patient C had MICA and EpCAM CD9 positive EVs.

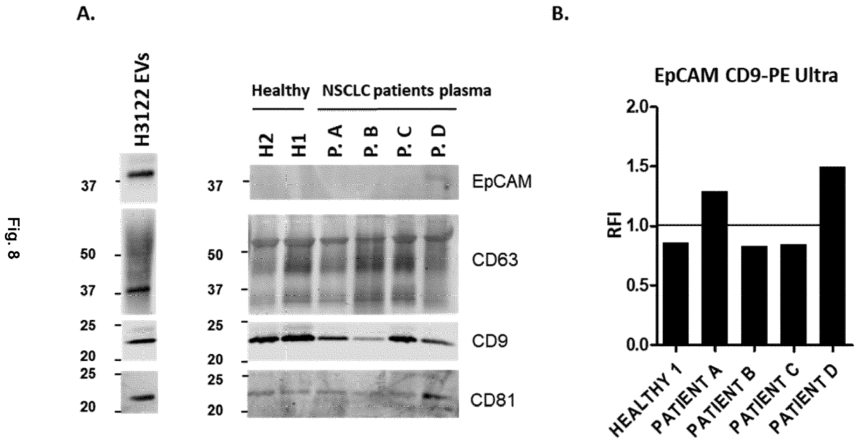

**Fig. 8. : Immunocapture followed by flow cytometry analysis of EVs is more sensitive than Western Blot analysis.**
1 ml of plasma from NSCLC patients (PATIENT A-D) and healthy donors (HEALTHY 1 and 2) were subjected to ultracentrifugation. 30 $\mu$l of EVs were analyzed in parallel. **A.** Western Blot: 25 $\mu$l of each EV preparation were loaded in a 12% SDS-PAGE gel. The membrane was immunoblotted for detection of : tetraspanins CD9, CD63, CD81 as general EV markers and EpCAM. An EV preparation from H3122 cell line was loaded in the same gel as a control for EpCAM positive EVs, the image of this lane was scanned at lower intensity than the rest of the lanes in the same blot and shown separately (left). **B.** Bead immunocapture followed by Flow Cytometry: 2 $\mu$l of each EV preparation were incubated during 18 hours with 3000 anti-EpCAM or IgG isotype control beads in 24 $\mu$l. EVs captured in each assay were detected by incubation with anti-CD9-PE. Both techniques showed comparable results, but flow cytometry was more sensitive.

## Examples

### Materials and Methods

#### Cells lines and reagents

Metastatic Melanoma cell lines Ma-Mel-55 and Ma-Mel-86c, (derived from melanoma patient metastases), provided by Prof. Annette Paschen (University Hospital of Essen, Germany), were described elsewhere (Ugurel S. et al. 2007, Chao F. et al. 2016, Lopez-Cobo Oncoimmunology). Lung cancer cell line H3122 (ATCC), was provided by Dr. Atocha Romero Alfonso (Hospital Universitario Puerta del Hierro) and satellite genotyped at the IIB-CSIC. Metastatic Melanoma and lung cancer cell lines were cultured in RPMI 1640 with 10% FBS, 1 mM L-Glutamine, 100 $\mu$g/ml Penicillin and Streptomycin, 1 mM Sodium Pyruvate, 0.1 mM non-essential aminoacids and 10 mM HEPES, at 37 °C. Lyophilized Exosome Standards from PC3 prostate cancer cell line cell culture supernatant (HansaBiomed or Immunostep S.L) were resuspended at 1 $\mu$g/$\mu$l for use. Unless otherwise stated, all chemicals were purchased from sigma.

#### Exosome extraction and isolation

Cells were cultured for 72 hours in a medium containing 1% exosome-free FCS (centrifuged at 100,000 x g for 20 h). Cell supernatants were centrifuged for 10 min at 200 x g and exosomes purified by sequential centrifugation as previously described (Ashiru, et al., 2010). Briefly, after centrifugation of cells, supernatants were centrifuged twice at 500 x g for 10 min. Supernatants were then centrifuged at 10,000 x g for 30 min prior to exosome isolation by ultracentrifugation at 100,000 x g for 2 hours at 4°C. Exosomes were resuspended in Hepes-buffered saline buffer (HBS: 10mM HEPES pH 7.2, 150mM NaCl) 0.22 $\mu$m filtered.
For sedimentation velocity analysis experiments, a further isolation step was performed to increase the purity of the preparation by size exclusion chromatography (SEC) through a 1 mL bed of Sepharose CL-2B (Sigma CL2B300) in HBS (pH 7.2, 0.22 $\mu$m filtered). The eluate was collected in fractions of 0.2 mL. For each fraction, the protein concentration was determined by measuring absorbance at 280 nm and the EV containing fraction was determined by anti-CD81 (MEM-38) dot blot. EV-enriched fractions were pooled and used for sedimentation rate analysis experiments.

Exosome quantitation

The concentration and size of purified exosomes in each sample were determined by nanoparticle tracking analysis (NTA) in a Nanosight NS500 (Malvern Instruments Ltd, Malvern, UK) equipped with a 405 nm laser. The settings used were: Camera level: 12, Focus between -15 and +15, Threshold: 10, Capture: 60 s., Number of Captures: 3, Temperature 25 °C. The experiments were carried out at the laboratory of Dr. H Peinado, Spanish National Centre for Oncological Research (CNIO).

Western Blot

EVenriched preparations were loaded in 12% SDS-PAGE gels, either under reducing or non-reducing conditions, as indicated in the experiments, and transferred to membranes with Trans-Blot® Turbo™ Transfer Packs (Biorad). Membranes were blocked using 5% non-fat dry milk in PBS containing 0.1% Tween 20 (PBS-T). Primary antibody was incubated for 1 h in PBS-T and, after washing, membranes were incubated with the appropriated secondary antibody. Primary antibodies used were: monoclonal anti $\beta$-actin produced in mouse (Sigma) at 0.13 $\mu$g/ml; purified mouse monoclonal anti-CD9 (MEM62), -CD63 (MEM259) and -CD81 (MEM-38) antibodies (kind gifts from Vaclav Horejsi, Croatia), Biotinylated anti-EpCAM (clone VU-1D9), biotinylated goat polyclonal anti-MICA antibody BAF13001 (R&D biosystems) and MelanA [Zytomed (M2-7C10 clone)]. Secondary antibodies used were Alexa-700 GAM or Alexa-790-SA (ThermoFisher) when proteins were visualized using the Odissey Infrared system (LI-COR Biosciences). When proteins were visualized using the ECL system (Amersham Biosciences) horseradish peroxidase-conjugated goat anti-mouse antibody (Dako) or horseradish peroxidase- streptavidin (Biolegend) were used.

Zeta potential

Ma-Mel-86c derived EVs obtained by ultracentrifugation at 100,000 x g were resuspended in isotonic HBS and lyophilized (at 2.2 x $10^9$ particles/$\mu$L, measured by Nanosight). After rehydrating and diluting to a final concentration of 3.4 x $10^7$ EVs/ml in PBS (in the range recommended for measurement in the instrument), EVs were treated with 8 $\mu$g/ml of cationic polymer hexadimethrine bromide (also known as Polybrene) or 1 $\mu$g/ml Poly-L-Lysine (a non-treated control sample was prepared in parallel as a control) and incubated for 5 min before diameter and Zeta potential measurement by Zetaview (Particle Metrix) a Nanoparticle Tracking Analysis (NTA) instrument.

DLS

Ma-Mel-86c derived EVs obtained by ultracentrifugation at 100,000 x g were resuspended in isotonic HBS and lyophilized (at 1.9 x $10^9$ particles/$\mu$L, measured by Nanosight). After rehydrating and diluting 1:1000 in HBS, EVs were treated with 8 $\mu$g/ml of cationic polymer Polybrene or 4 $\mu$g/ml Poly-L-Lysine (a non-treated control sample was prepared in parallel as a control) and incubated for 5 min or 18h before diameter measurement by Dynamic Light Scattering (DLS) using a NanoZS (Red badge) ZEN3600 equipped with a 633 nm laser. Experiments were carried out at the Instituto de Ciencia de Materiales de Madrid - ICMM - CSIC

Sedimentation rate analysis

Two different EV preparations derived from the melanoma cell line Ma-Mel-86c were used for analytical centrifugation experiments: a sample obtained by ultracentrifugation of conditioned medium (2.6 x $10^9$ particles/$\mu$L) was diluted 1:10 in HBS; a second sample obtained by size exclusion chromatography (SEC) was used without dilution. For each type of sample 3 different experiments were run to compare different conditions: 1) Polybrene at a final concentration of 4 $\mu$g/ml; 2) Poly-L-lysine at a final concentration of 4 $\mu$g/ml, and 3) control with no polymer added. After an 18-hour incubation all samples were run in an analytical ultracentrifuge (XLI, wavelength: 280, 3000 krpm). The results were analyzed with SEDFIT 16.1c analysis Software with a confidence interval variation of 0.68. The experiment was carried out at the Servicio de Interacciones Moleculares. Centro de Investigaciones Biologicas, C.S.I.C.

Sample pre-treatment for direct exosome detection in biological samples

Plasma samples, were centrifuged for 10 min at 500 x g, followed by a 2000 x g centrifugation for 10 min, to eliminate cells or cell debris in biological samples.

Antibody coated magnetic beads preparation

Antibody-coated magnetic beads were obtained from the Exostep™ kit (Immunostep, Ref: ExoS-25-U) or prepared by incubating magnetic fluorescent beads either from Luminex (MagPlex Microsphere, Ref: MC10012) or Bangs (QuantumPlex M SP Carboxil, Ref: 251A) with purified anti-CD63 (Clone TEA3/18), purified anti-CD9 (Clone VJ1/20) or anti-EpCAM (Clone VU-1D9) antibodies from Immunostep, S.L or anti-MICA (R&D) or IgG isotype control (Sigma). Magnetic fluorescent beads have high density of carboxyl functional groups on the surface, these groups were used to covalently conjugate antibodies through primary amines by two-step EDC/NHS coupling producing a stable amide bond (Campos-Silva et al., 2019, Scientific Reports 9:2042, 1-12).

EV detection by Flow cytometry

EVs were incubated with 3000 antibody-coated beads in either 100 $\mu$L, 50 $\mu$L or 12 $\mu$L of PBS containing 1% casein (EV enriched preparations diluted 1:100-1:1000) for 18 h, in either a 5 ml tube or a well in a 96 wells plate, as specified in each experiment, without agitation at room temperature (RT). When using biological samples, 12 $\mu$L of pretreated samples were added to the 12 $\mu$L of beads in PBS containing 1% casein. Background signals were determined by comparison with antibody isotype-coupled beads. When adding cationic polymers, they were added and mixed before the 18 hours incubation, unless otherwise stated, at a final concentration of 4 $\mu$g/ml of Polybrene or Poly-L-lysine, controls were incubated in the same volume of PBS without polymer. After the capture step, beads were washed with filtered PBS containing 1% casein, and, and recovered using a Magnetic Rack (MagneSphere(R) Mag. Sep. Stand 12-hole, 12 $\times$ 75 mm Promega, Ref Z5343 or 40-285 Millipore Handheld Magnetic Separation Block for 96 well plates).The recovered beads were stained with either anti-CD63 (Clone TEA3/18), anti-CD81 (MEM-38) or anti-CD9 (Clone VJ1/20) antibodies (Immunostep, S.L.), either biotinylated or directly conjugated to PE. After antibody binding, beads were washed with filtered PBS containing 1% casein, and recovered using the Magnetic Rack. When using biotinylated antibodies, a further step incubating with streptavidin-PE (Immunostep, S.L.) was necessary for detection, followed by bead washing with PBS-1% casein. 1500-2000 events were acquired by flow cytometry using Gallios or Cytomics FC 500 (Beckman Coulter) or CytoFLEX (Beckman Coulter) and data were analyzed using Kaluza (Beckman Coulter) or FlowJo (Tree Star, Inc) software. Single beads were gated in Forward Scatter in the region corresponding to 6 $\mu$m (established using calibration beads (FlowCheck ProTM fluorospheres, Beckman Coulter), excluding bead doublets and selecting APC-positive events.

ELISA

MICA, EpCAM and tetraspanins were detected in lung cancer or melanoma-derived exosomes using sandwich ELISA. Plates were coated with purified mAb R&D against MICA (MAB13002), purified mAb Immunostep S. L. against EpCAM (326PU-01MG) or antitetraspanin antibodies (anti-CD9 VJ1/20 or anti-CD63 Tea3/18 from Immunostep S. L.) in BBS (Borate Buffered saline) or IgG (MOPC-21) as negative control, overnight at 4°C. After blocking the plates with 1% casein-PBS for 2 h at 37°C, samples were added: either the EV enriched preparation diluted 1:100-1:1000 in HBS or plasma diluted ½ in 1% casein-PBS and incubated 18 hours at RT. When adding cationic polymers, they were mixed with the sample before the 18-hour incubation.. Unless otherwise stated, polybrene and poly-L-lysine were added at a final concentration of 4 $\mu$g/ml. HBS was added to controls to maintain the same final volume. Biotinylated secondary antibodies [CD9 VJ1/20 or CD81 M38 from Immunostep S. L.] were added and followed by streptavidin-HRP (Amersham). The reaction was developed using TMB (3,3',5,5'-Tetramethylbenzidine) substrate (1-Step™ Ultra TMB-ELISA Substrate Solution; Thermo). Absorbance was measured at 450 nm with Multiskan™ FC Filterbased Microplate Photometer (Thermo Scientific™).

Transmission Electron Microscopy

For Transmission Electron Microscopy (TEM), 1 $\mu$L Ma-Mel-55 melanoma-derived EV preparation (0.6 x $10^9$ particles /$\mu$L) were diluted 1:10 in HBS. Polymers were added at a final concentration of 4 $\mu$g/ml of Polybrene or Poly-L-lysine. After an 18-hour incubation, samples were floated on carbon-coated 400-mesh 240 Formvar grids, incubated with 2% uranyl acetate, and analyzed using a Jeol JEM 1011 electron microscope operating at 245 100 kV with a CCD camera Gatan Erlangshen ES1000W. The experiment was carried out at the Electron Microscopy Facility, Spanish National Centre for Biotechnology (CNB).

Example 1 - Results

Standard antibody binding conditions are not 100% efficient for the capture of extracellular vesicles

Recently, the inventors disclosed a high sensitivity method for immunocapture and detection of EVs by flow cytometry, said method being based on the use of antibody-coated beads followed by detection with a labelled antibody, both recognizing different epitopes on the EVs (Campos-Silva et al., 2019, Scientific Reports 9:2042, 1-12). During the optimization of that method, the inventors calculated the theoretical numbers of EVs that would bind the antibody-coated microspheres and analyzed the saturation curve in experiments with increasing amounts of EVs. While 6000 beads could theoretically bind $3.85 \times 10^7$ EVs, the experiments showed saturation of detection when around $3.6 \times 10^9$ EVs were offered, suggesting that the beads were not binding all the EVs that were present in the incubation mix. Several explanations could be proposed. Firstly, since EVs are usually a heterogeneous mix, it could be possible that not all the EVs in the mixture contained the epitope for the capture antibody. To investigate whether any of the unbound EVs contained the epitope for the capture antibody, nested incubations were carried out incubating the supernatant from a previous experiment with fresh beads. After several rounds of capture using beads coated with the same antibody, EVs were still detected in supernatants (Fig. 1). Characterization of EVs is shown in Fig. 2. These experiments demonstrate that, during the capture step, a large number of vesicles were not binding to the beads, due to a limitation in the methodology and not because of the lack of an epitope. Because agitation did not improve the efficiency of binding, and a long incubation time was crucial for good detection, we decided to explore whether a more efficient capture of EVs could be achieved by modulating their physico-chemical properties. Due to their nanometric size, solutions of EVs could be considered colloidal suspensions, in fact, methods for colloids are often used to characterize EV preparations. Thus, experiments to analyze the putative colloidal behavior of EVs were carried out including study of their stability/flocculation properties.

EVs in solution, as stable colloidal suspensions, are destabilized using charged polymers

Polymers are broadly used in the industry to clarify liquids containing particles in suspension. The same phenomenon is sometimes used in biology for very different applications, for example, transfection using viral vectors. Although it was initially proposed that the enhancement of transfection with the addition of polymers was based on the neutralization of charges, it has since been demonstrated that charged polymers affect the nature and intensity of the driving forces for virus aggregation, sedimentation and adsorption (Davis et al., 2004 Biophys J 86(2): 1234-1242). Therefore, transfection using viral particles and polymers is yet another example of flocculation within a complex colloidal system. In order to evaluate the effect of depletion forces and other biophysical properties in EV suspensions, several parameters were studied after incubation with two polymers normally used in biology for precipitation of nanometric structures: hexadimethrine bromide (Polybrene) and Poly-L-Lysine. First, the diameter and zeta-potential of metastatic melanoma derived EVs obtained by ultracentrifugation were measured by NTA (ZetaView) (Table 1) and Dynamic Light Scattering (DLS) and Fig. 3 A). -Melanoma-derived EVs have a negative Zeta-potential and this parameter can be used as an approximation to evaluate colloidal stability because electrostatic repulsion prevents aggregation. By NTA, melanoma-derived EVs in PBS had a diameter of 138.6 nm and a negative Zeta-potential of -9.50 mV. When EVs were incubated with either 8 $\mu$g/ml Polybrene or 1 $\mu$g/ml Poly-L-Lysine for 5 min, the zeta-potential of EVs changed from a negative to a positive net charge ranging between $+9.27 \pm 0.40$ mV and $15.05 \pm 0.17$ mV in the different conditions.

Table 1: Diameter and Z-potential values of EVs from metastatic melanoma cell lines

| SAMPLE | Original Concentration (particles/ml) | Diameter (nm) | Z potential |
|---|---|---|---|
| Ma-Mel-86c EVs in PBS | 2.2E+12 | 138.6 | -9.50 $\pm$ 0.40 mV |
| Polybrene 8 $\mu$g/ml in PBS | 3.2E+7 | 97.4 | 15.05 $\pm$ 0.17 mV |
| Ma-Mel-86c EVs with 8 $\mu$g/ml Polybrene in PBS-B | 2.5E+12 | 116.4 | 9.27 $\pm$ 0.41 mV |
| Poly-L-Lysine 1 $\mu$g/ml in PBS | 7.0E+7 | 210.2 | 12.22 $\pm$ 0.06 mV |
| Ma-Mel-86c EVs with 1 $\mu$g PolyL in PBS-A | 4.1E+12 | 243.9 | 10.04 $\pm$ 0.05 mV |

Since the diameter and charge data obtained by light scattering methods suggested that polymers could cause aggregation and flocculation of EVs, analytical ultracentrifugation was used to measure the sedimentation rate of nanoparticles in the presence of positively charged polymers (Fig. 3 B). The average sedimentation coefficient of the EV suspension, either obtained by ultracentrifugation or SEC, increased dramatically in the presence of the charged polymers. Interestingly, after an 18-hour incubation with cationic polymers, the sample presented high polydispersity with multiple peaks displaying a wide range of sedimentation coefficients (not shown). This implies that larger particles are present in the polymer-treated sample compared to control samples. In line with these observations, electron microscopy imaging of EVs after an 18-hour incubation with cationic polymers showed large high electron dense spots (1-2 $\mu$m), consistent with high mass particles (Fig. 3 C). When obtaining more detailed images, clusters of EVs at that sites could be observed, in fact inspection of the grids at low magnification already showed the high density masses. In fact, when EVs were incubated with poly-L-Lysine, the electron beam ruptured the resin supporting the sample and high magnification images could not be obtained. It has previously been shown in virus containing solutions that high molecular weight poly-L-Lysine (up to 300 KDa) can cause relatively higher aggregation than the lower molecular mass polymer polybrene (4-6 kDa) (Davis et al., 2004 Biophys J 86(2): 1234-1242).

Taken together, these results indicate that when cationic polymers are added to EV solutions, clusters of particles of different sizes are generated leading to higher sedimentation rates. This phenomenon perfectly fits with the model of polymer-induced colloidal flocculation, in which particle aggregation most likely occurs by 1) neutralization of charge caused by patches of adsorbed polyelectrolyte and/or 2) formation of bridges between colloidal particles by simultaneous adsorption of polyelectrolyte chains onto more than two particles (Zhou and Franks, 2006 Langmuir 22, 16, 6775-6786). Colloidal stability depends on several phenomena which include electrostatic stabilization, steric stabilization and depletion forces.

Cationic polymers destabilize EV suspensions increasing the detection of scarce proteins on EVs

Since cationic polymers destabilized EV suspensions, increasing the average diameter of the particles and the sedimentation coefficient, we hypothesized that a depletion force phenomenon described for colloidal systems would also be involved in EV preparations allowing better detection in immuno-capture methods. Thus, we explored next how the inclusion of cationic polymers affected the precipitation of nanoparticles on antibody-coated surfaces either in bead-assisted flow cytometry or ELISA experiments. For these experiments, EVs obtained by ultracentrifugation from the lung cancer cell line H3122 were incubated with cationic polymers and analyzed for their expression of EpCAM. The characterization of these vesicles was performed first by classical methods including NTA, Western Blot and EM to establish the markers present in EVs derived from different tumor cell lines (Fig. 2). Initial polymer titration experiments (Fig. 4) revealed that: 1) cationic polymers did not interfere with immunodetection assays; 2) the inclusion of cationic polymers provided a clear advantage when the capture antibody was directed to a scarce protein, such as the tumor associated protein EpCAM in H3122 lung cancer cell line derived EVs, and capture recovery increased sensibly.

The unique characteristics of colloids might explain the need of flocculants to allow binding between antibody coated microparticles and exosomes. Microparticles can precipitate with time due to gravity. However, because the mass of each dispersed nanovesicle is very small, gravity and buoyancy forces are not sufficient to counteract Brownian motion and EVs remain in suspension in the mix. The differences in their behavior in suspension makes it statistically difficult that both types of particles interact. Adding flocculants would allow the exosomes to precipitate in a controlled manner allowing the interaction with antibody coated microparticles or surfaces.

Optimized immunocapture of EVs allows direct detection of tetraspanins and tumor associated proteins in plasma

The experiments shown above demonstrate that EV suspensions require an external driving force to allow precipitation of nanoparticles onto antibody-coated surfaces during immunocapture experiments. To optimize the protocol and increase EV binding on beads, new experiments were designed to facilitate the encounter of microparticles and nanometric EVs during the capture step (Fig. 5). Using conditions of minimal volume, the detection of tetraspanins increased extraordinarily in experiments using EVs from cell lines obtained by ultracentrifugation, suggesting that this method would have the sensitivity required to detect EVs directly in plasma.

To confirm that the optimized immune-capture method allowed the detection of physiological concentrations of EVs in healthy donor's plasma, antibodies against tetraspanins were used both for capture and detection in flow cytometry analysis. Indeed, EVs were efficiently captured and detected in plasma without any previous EV enrichment procedure using as little as 12 $\mu$L of plasma (Fig. 6 A). Beads coated with an irrelevant antibody (IgG isotype) were used in parallel as a negative control. The sensitivity of the assay was very high implying that probably tumor-derived antigens, which are usually less abundant than tetraspanins on EVs, could also be detected using this method. To check whether tumor derived antigens in EVs could be detected directly in plasma using this methodology, H3122-derived EVs (positive for EpCAM) were added to a healthy donor plasma sample (at a final concentration of 6,25 x 107 EVs/$\mu$l) and the immunocapture experiment was performed. While healthy donor plasma without lung cancer EVs added was negative for EpCAM, the same sample showed a high positive signal for anti-EpCAM beads, as well as an increased signal for tetraspanins, when the EVs were added (Fig. 6 A). In order to estimate the amount of EVs that could be recognized using this method, a titration experiment was performed. The sensitivity of the assay was high enough to detect tumor associated antigens, such as EpCAM from H3122 lung cancer-derived EVs, at a concentration of 3.125 x 106 EVs/$\mu$l (Fig. 6 B).

Minimal volume of plasma from lung cancer patients is enough to detect EV-bound EpCAM

To validate the method of detection of tumor antigens directly in plasma from cancer patients, 12 $\mu$L of plasma from four Non-Small Cell Lung Cancer patients (Patients A-D) and 2 healthy donors were incubated for 18 hours with beads coupled to anti-MICA, anti-EpCAM or anti-CD63 (1:2 dilution). EVs captured in each assay were detected in parallel by either anti-CD81-PE (A) or anti- CD9-PE (B) (Fig. 7). Patient A presented MICA- and EpCAM-CD81 positive EVs and Patient C presented MICA- and EpCAM-CD9 positive EVs. This experiment demonstrates that this optimized methodology allows tumor antigens associated to EVs detection directly in plasma from cancer patients.

Plasma EVs immunocapture followed by Flow cytometry shows comparable results but higher sensitivity than Western Blot.

Finally, we compared the results obtained by flow cytometry of direct immunocapture of EVs from plasma with the results obtained by EV characterization by Western Blot. A 1 ml fraction from plasma was subjected to ultracentrifugation and the EV-enriched preparation was resuspended in 30 $\mu$l; both techniques were tested using the same preparation, however, different volumes were used: 25 $\mu$l were analyzed by Western Blot while only 2 $\mu$l were analyzed by flow cytometry after incubation with anti-EpCAM beads, showing comparable results (Fig. 8 A and B). After ultracentrifugation, plasma EVs from patient D presented the highest signal for EpCAM both in flow cytometry and Western Blot. These data demonstrate that flow cytometry has higher sensitivity than Western Blot.

When comparing the characterization of plasma EVs in the non-processed sample and after ultracentrifugation, several differences were observed. Patient A had CD9+EpCAM+ EVs in both samples, however, it was only detectable by flow cytometry and not by WB, suggesting that they were below the limit of detection for WB. CD9+EpCAM+ EVs were detected in the unprocessed serum sample of patient C but not after ultracentrifugation. Patient D only presented EpCAM+ EVs in the ultracentrifuged sample. These data imply that ultracentrifugation can select different EV subpopulations from the original plasma sample.

Conclusions

The biological, physiological and pathological roles of EVs discovered in the last decades have encouraged researchers to characterize their quantity and phenotype in biological samples obtained from patients and, more importantly, to validate their use as biomarkers in large cohort of patients. Classical EV characterization techniques are not easily translatable to the clinics. To solve the problems in carrying out large-scale screening projects in any research or clinical setting in the field of exosomes, the development of broadly used techniques which allow the identification of vesicles in small volumes and with minimal sample manipulation is necessary. An extra level of complications arises from the fact that any biological fluid contains exosomes from many cellular origins and samples can be very heterogeneous.

With this idea in mind, we and others have proposed several immune detection-based techniques for EV characterization, including ELISA or lateral flow immunoassay (LFIA) (Oliveira-Rodriguez et al., J Extracell Vesicles 5: 31803, 1-11). However, the sensitivity of these assays usually is not enough to establish a simple protocol that would not need a large biological sample volume or time-consuming biological sample processing for EV enrichment. For these reasons, clinical laboratories have not implemented such studies. The recent development of nanosensors has also provided an excellent tool to design studies in a large number of patients (Jeong, S. et al. 2016, ACS Nano 10(2): 1802-1809; Im, H et al. 2017 Lab Chip 17(17): 2892-2898). These devices allow the use of small amounts of samples and specific detection of exosomes. However, they require purpose-designed devices built-up in specialized laboratories and usually complex reading instruments, thus, studies based on these methodologies are still scarce.

Flow cytometry has been widely used for the analysis of cellular proteins, and it is basic in clinical studies and blood-based diagnostics. Therefore, much effort has been invested to adapt this method to phenotype EVs. Since EV size is below the limit of resolution of conventional flow cytometers, several approaches have been developed based on binding EVs to different types of spheres (latex, polystyrene, different sizes and functionalization) for EV visualization by conventional flow cytometers. However, most of these protocols can only be performed in research laboratories because they still require a large sample volume, laborious procedures of nanovesicle pre-enrichment and the use of sophisticated techniques or specialized personnel.

The method presented here provides an optimization of these procedures to achieve the required sensitivity and overcome the problems explained above. The new experimental conditions will allow phenotyping proteins present on the surface of EVs and, more importantly, to perform systematic analyses on large numbers of patient samples. With this method, in which the volume of starting material is minimal, and a previous isolation or enrichment step is not required, EV detection radically improves in immune-capture assays. The protocol takes 2-3 hours hands-on and has a high sensitivity (with a limit of detection of down to $10^6$ EVs/$\mu$l in plasma).

Based on the colloidal nature of EVs we have demonstrated that the addition of polymers to EV suspensions leads to the precipitation of vesicles allowing a more efficient antibody capture in both ELISA assays and bead-based assays. This methodology will open the way for the study of large panels of samples with minimal effort in any laboratory setting.

**Claims**

1. A method for the detection and/or quantification of extracellular vesicles (EVs) from a biological fluid sample comprising:

   a) Obtaining a biological fluid sample of volume between 5 and 45 $\mu$L;
   b) Contacting the biological fluid sample obtained in a) to a solid surface functionalized with at least one capturing molecule capable of binding EVs, obtaining a first solution;
   c) Incubating the first solution obtained in step b) with at least one detection molecule capable of binding to an epitope in the EVs; and
   d) Detecting and/or quantifying the detection molecule from step c) bound to the epitope of EVs.

2. The method according to claim 1, wherein a cationic polymer is added to the biological fluid sample obtained in step a).

3. The method according to claims 1 or 2 wherein the biological fluid sample is selected from the list consisting of: urine, serum, plasma, amniotic liquid, cerebrospinal fluid, ascites, bronchoalveolar wash, synovial liquid, breast milk, saliva, bone marrow and blood.

4. The method according to any one of claims 1 to 3 wherein the capturing molecule functionalized to the solid surface and/or the detection molecule is selected from a list consisting of: annexin, lectin and immunoglobulin; wherein preferably the capture molecule and the detection molecule are different.

5. The method according to claim 4 wherein the immunoglobulin is an antibody selected from the list consisting of: anti-CD63, anti-CD81, anti-CD9, anti-tetraspanins, anti-EpCAM and anti-MICA antibody; the annexin is selected from a list consisting of: annexin V, A1, A2, A4 and A5; and the lectin is selected from a list consisting of: *Solanum tuberosum* lectin, *Sambucus nigra* agglutinin lectin, *Sambucus sieboldiana* agglutinin lectin, *Trichosanthes japonica* agglutinin II lectin, galectin 1 and galectin 3.

6. The method according to any one of claims 2 to 5 wherein the cationic polymer added to the biological fluid sample is selected from the list consisting of: poly-L-lysine, hexadimethrine bromide, gelatin, acrylamide chloride, diallyldimethylammonium chloride, cationic chitosan, cationic cellulose, cationic cyclodextrin, polyethyleneimine, polyamidoamines, polyamino-co-ester, polydimethylaminoethylmethacrylate and cationic dextran, preferably poly-L-lysine and hexadimethrine bromide.

7. The method according to any of claims 1 to 6 wherein the solid surface are microparticles, preferably polymer microparticles, latex microparticles or magnetic microparticles.

8. The method according to any one of claims 1 to 7 wherein the biological fluid sample is obtained from a mammal, preferably a human.

9. The method according to any one of claims 1 to 8 wherein the detection in step d) is made by a laser-based detection instrument or flow cytometry, preferably flow cytometry.

10. The method according to any one of claims 1 to 9 wherein the EVs are exosomes.

11. The method according to any one of claims 1 to 10 wherein the detection molecule of step c) is conjugated to a detectable label.

**12.** A method for the detection and/or identification of biomarkers of a disease in EVs which comprises the steps of the method according to any one of claims 1 to 11, and an additional step (e) in which disease biomarkers in EVs are phenotyped.

**13.** An in vitro method for the diagnosis of a disease which comprises the steps of the method according to any one of claims 1 to 11, wherein the capturing molecule capable of binding EVs in step b) binds to a disease biomarker or the epitope in the EV from step c) is an epitope of a disease biomarker and the binding by the capturing molecule to a disease biomarker in the step b) or the binding by the detection molecule to an epitope in the EV from step c) is indicative of disease.

**14.** The method according to claim 12 or the method according to claim 13 wherein the disease is selected from the list consisting of: cancer, autoimmune diseases, inflammatory diseases, immunodeficiencies, infectious diseases, pathogen originated diseases, cardiovascular diseases, degenerative diseases, hormone disorder related diseases, and metabolic diseases.

**15.** The method according to claim 14, wherein the disease is cancer, Alzheimer's disease or diabetes.

A.

Legend:
☐ IgG - 2 µg PC3 EVs
■ CD63 - 2 µg PC3 EVs
▨ CD63 - SN 24 h
▨ CD63 - SN 48 h
☐ CD63 - SN 72 h

Count (y-axis): 0, 10, 20, 30
PE (x-axis): $10^3$, $10^4$, $10^5$, $10^6$

B.

| Sample | RFI |
|---|---|
| CD63 – 2 µg PC3 EVs | 6.27 |
| CD63 - SN 24 h | 4.55 |
| CD63 - SN 48 h | 3.69 |
| CD63 - SN 72 h | 1.68 |

Fig. 1

EP 3 936 864 A1

Fig. 2

## A. NTA

**Ma-Mel-86c** — Concentration (particles /ml) vs (nm)

| Average φ | 196.5 +/- 1.4 nm |
|---|---|
| Mode φ | 157.4 +/- 3.7 nm |
| Particles/µL | $1.22 \cdot 10^9$ +/- $1.09 \cdot 10^8$ |

**PC3** — Concentration (particles /ml) vs (nm)

| Average φ | 166.8 +/- 1.5 nm |
|---|---|
| Mode φ | 116.3 +/- 4.3 nm |
| Particles/µL | $6.55 \cdot 10^8$ +/- $3.04 \cdot 10^7$ |

**H3122** — Concentration (particles /ml) vs (nm)

| Average φ | 244.2 +/- 6.1 nm |
|---|---|
| Mode φ | 139.2 +/- 3.1 nm |
| Particles/µL | $4.19 \cdot 10^8$ |

Fig. 2 (continuation)

Fig. 3

C.

**Untreated**   **Polybrene**   **Poly-L-Lysine**

0.5 µm

0.5 µm

Fig. 3 (continuation)

**B.**

BLANK
EpCAM
CD63

OD (450 nm)

| OD_test−OD_blank | EpCAM | CD63 |
|---|---|---|
| Untreated | 0.1 | 0.15 |
| Polybrene | 0.11 | 0.23 |
| Poly-L-Lysine | 0.15 | 0.26 |

**A.**

RFI

| | RFI | SI |
|---|---|---|
| Untreated | 7.11 | 78890.09 |
| Polybrene | 9.13 | 90495.86 |
| Poly-L-Lysine | 12.09 | 117947.6 |

Fig. 4

23

Fig. 5

A.

B.

Fig. 6

Fig. 7

Fig. 7 (continuation)

Fig. 8

Europäisches
Patentamt

European
Patent Office

Office européen
des brevets

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/066589 A1 (CARIS LIFE SCIENCES LUXEMBOURG HOLDINGS [LU] ET AL.) 3 June 2011 (2011-06-03) | 1,3-5, 7-15 | INV. G01N33/50 G01N33/543 |
| Y | * abstract * <br> * examples 1-22 * <br> * claims 1-38 * | 2,6 | |
| Y,D | WO 2017/198695 A1 (UNICYTE EV AG [CH]) 23 November 2017 (2017-11-23) * abstract * * claims 1-15 * * Example * | 2,6 | |
| Y | WO 2017/178472 A1 (UNICYTE EV AG [CH]) 19 October 2017 (2017-10-19) * abstract * * page 10 - page 16 * * claims 1-12 * | 2,6 | |

TECHNICAL FIELDS
SEARCHED (IPC)

G01N

The present search report has been drawn up for all claims

| Place of search | Date of completion of the search | Examiner |
|---|---|---|
| Munich | 27 November 2020 | Giry, Murielle |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 38 2602

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2020

| Patent document cited in search report | | Publication date | Patent family member(s) | | Publication date |
|---|---|---|---|---|---|
| WO 2011066589 | A1 | 03-06-2011 | AU | 2010324594 A1 | 14-06-2012 |
| | | | CA | 2782284 A1 | 03-06-2011 |
| | | | EP | 2507393 A1 | 10-10-2012 |
| | | | US | 2013203061 A1 | 08-08-2013 |
| | | | WO | 2011066589 A1 | 03-06-2011 |
| WO 2017198695 | A1 | 23-11-2017 | EP | 3246703 A1 | 22-11-2017 |
| | | | EP | 3458854 A1 | 27-03-2019 |
| | | | US | 2020182865 A1 | 11-06-2020 |
| | | | WO | 2017198695 A1 | 23-11-2017 |
| WO 2017178472 | A1 | 19-10-2017 | CN | 109153968 A | 04-01-2019 |
| | | | EP | 3443112 A1 | 20-02-2019 |
| | | | JP | 2019518428 A | 04-07-2019 |
| | | | US | 2020179827 A1 | 11-06-2020 |
| | | | WO | 2017178472 A1 | 19-10-2017 |

EPO FORM P0459

For more details about this annex : see Official Journal of the European Patent Office, No. 12/82

EP 3 936 864 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013158203 A
- WO 2018070479 A
- WO 2017198695 A

### Non-patent literature cited in the description

- **CHAO F. et al.** *Lopez-Cobo Oncoimmunology,* 2016
- **CAMPOS-SILVA et al.** *Scientific Reports,* 2019, vol. 9 (2042), 1-12
- **DAVIS et al.** *Biophys J,* 2004, vol. 86 (2), 1234-1242
- **ZHOU ; FRANKS.** *Langmuir,* 2006, vol. 22 (16), 6775-6786
- **OLIVEIRA-RODRIGUEZ et al.** *J Extracell Vesicles,* vol. 5 (31803), 1-11
- **JEONG, S. et al.** *ACS Nano,* 2016, vol. 10 (2), 1802-1809
- **IM, H et al.** *Lab Chip,* 2017, vol. 17 (17), 2892-2898